# EUROPEAN PATENT SPECIFICATION

(11) **EP 1 494 629 B1**
(45) Date of publication and mention of the grant of the patent: **14.02.2007**
(21) Application number: 03720700.8
(22) Date of filing: 14.04.2003
(51) Int. Cl.: A61F 6/04, A61K 9/70

(54) **CONDOM**
KONDOM
PRESERVATIF

(30) Priority: 16.04.2002 GB 0208704
(43) Date of publication of application: 12.01.2005
(73) Proprietor: Futura Medical Developments Limited, Guildford Surrey GU2 5YG (GB)
(72) Inventor: BARDER, James Henry, Slaugham, West Sussex RH17 6AG (GB)
(74) Representative: Harrison, Ivor Stanley
(86) International application number: PCT/GB2003/001586
(87) International publication number: WO 2003/088880

(56) References cited:
- WO-A-00/13664
- WO-A-02/00240
- WO-A-02/39945
- WO-A-98/27899
- WO-A-99/21562
- WO-A-99/56666
- US-A- 4 829 991
- US-A- 5 333 621
- US-A- 6 000 398
- US-B1- 6 182 661

## Description

This invention relates to condoms and is particularly intended to provide a condom for provision of sexual stimulation to the female partner of the user, in order to alleviate female sexual dysfunction, to enhance sexual pleasure and to improve condom safety through lowering condom failure due to rupture, by increasing vaginal secretion and lubrication.

It is recognised that female sexual dysfunction is a complex condition which, due to its various causes, cannot readily be treated by use of a particular drug or device. Nevertheless, an increase in vaginal blood flow and clitoral engorgement is known to be associated with increased vaginal secretions, a decrease in dyspareunia and increases in clitoral sensation, orgasmic response and sexual desire. It is an object of the present invention, therefore, to provide a means of stimulating the female genitalia during intercourse to increase vaginal blood flow.

A condom according to the preamble of claim 1 is known from WO 02/00240.

In one aspect, the invention provides a condom having applied to its external surface a vasodilator active compound.

The vasodilator active compound is preferably disposed and retained in a localised region towards the open end of the condom whereby, in use during intercourse, the compound makes contact with the vaginal meatus or proximal region of the vagina, such that the vasodilator is absorbed through the lining of the vagina to stimulate and increase the flow of blood in the labia and through the clitoris to promote engorgement thereof which, itself, will lead to further or enhanced stimulation and result in increased vaginal secretions. Symptoms associated with female inorgasmia will thus be alleviated.

By use of the invention, a further advantage is that increased vaginal secretions result in increased lubrication between the wall of the vagina and the condom resulting in a lower rate of condom failure due to rupture.

In condoms according to the present invention, the active compound may be contained or impregnated in or coated on the external surface of the condom. The vasodilator may be applied as a composition which includes a carrier material with which the vasodilator compound is miscible but which will release the vasodilator active compound when in contact with body tissue. Where the condom is coated with a lubricant, the vasodilator active compound may be dispersed or dissolved in the lubricant but preferably is disposed on the condom surface in a form or within a composition which is immiscible with the lubricant, whereby the compound is localised substantially at the zone of application to the condom surface. The active compound on such condoms, when they are in their rolled-up state for packaging purposes, thus tends to resist translocation from the external surface to adjacent portions of the internal surface, whereby the active compound is retained predominantly on the external surface and within the original zone of application. Preferably, the lubricant is buffered to an acidic pH, for example between 3 to 5, to prevent hydrolysis of the vasodilator active compound.

Condoms according to the present invention may include a textured or undulating region to the external surface, to provide enhanced physical stimulation to the female genitalia during intercourse to increase sexual sensation. The textured or undulating region may be located towards the open end of the condom so that the proximal parts of the vagina and the clitoris are preferentially stimulated by contact with the texturing or undulations. The textured or undulating region preferably incorporates or includes the vasodilator active compound. The texturing or undulations may comprise ribs or an array of individual protrusions or may comprise merely a roughened surface region, formed either by imparting a pattern to the surface of the condom or by application to the surface thereof of a particulate material or a material having a high coefficient of surface friction, such as a highly plasticised elastomer. The textured surface may be formed by manufacturing the condom on a mandrel or mould having an appropriate pattern of ribs, dots or other texturing etched into or embossed on its surface or by applying to the condom a material which results in a textured surface, for example by extruding a thin stream of the material from a nozzle so as to form a series of rings around the condom or by spraying the material in such a way that the surface becomes textured. A suitably textured extruded section can be applied direct to the condom at the time of its manufacture or subsequently, either by direct bonding or by the use of a pressure-sensitive, hot-melt or other suitable type of adhesive. Alternatively, a coating of a material can be applied for example by spraying, the coating then having a texturing applied to it by contact with a suitably patterned die so as to mould the surface in the desired textured shape.

The textured surface may be formed from one or more layers of material including the material from which the condom itself is formed. The material of at least one such layer should be miscible with the vasodilator and should allow the vasodilator to be absorbed by skin or tissue when brought in contact with the condom. Preferably, the material from which the condom is formed, either natural rubber latex or a synthetic rubber-like material, and any lubricant used therein, is immiscible with the vasodilator or the vasodilator-containing composition, whereby the vasodilator is restrained from migrating to other parts of the condom other than the zone of application. The material from which the vasodilator-containing layer is formed will depend on the nature of the vasodilator active compound but, for active compounds such as organic nitrates, for example glyceryl trinitrate, suitable materials would include polar elastomers applied to the condom from solution, in the form of an aqueous dispersion of latex or by a hot melt or reactive process. Alternatively, the vasodilator-containing layer can be pre-formed and bonded subsequently to the condom using a suitable adhesive system as necessary.

The vasodilator active compound may comprise any known erectogenic compound which, on absorption through the skin or mucosa, locally enhances blood flow. Such compounds may include nitrates, long and short acting alpha-adrenoceptor blockers, ergot alkaloids, anti-hypertensives and the prostaglandins. Phosphodiesterase inhibitors, particularly type III and IV and most particularly type V can also be used, either alone or in combination with other vasodilators. Such compounds can be used alone or in combination and, optionally, together with skin penetration enhancers such as azone, dimethylsulfoxide, dimethyl formamide, N,N-dimethylacetamide, declymethylsulfoxide, polyethylene glycol monolaurate, glycerol monolaurate, lecithin and 1-substituted azacyclopheptan-2-one.

Useful nitrates and similarly acting compounds include nitro-glycerine, isosorbide dinitrate, erythrityl tetranitrate, amyl nitrate, sodium nitroprusside, molsidomine, linsidomine chlorydrate ("SIN-1"), S-nitroso-N-acetyl-d,1-penicillamine ("SNAP"), S-nitroso-N-cysteine, S-nitroso-N-glutathione ("SNO-GLU") and diazenium diolates ("NONOates"). A particularly useful nitrate is nitro-glycerine.

Natural prostaglandins that can be used include PGE₀, PGE₁, PGA₁, PGB₁, PGF₁alpha, 19-hydroxy-PGA₁, 19-hydroxy-PGB₁, PGE2, PGA₂, PGB₂, 19-hydroxy-PGA₂, 19-hydroxy-PGB₂, PGE₃, PGF₃alpha. Semi synthetic and synthetic prostaglandins such as carboprost tromethamine, dinoprost tromethamine, dinoprostone, lipoprost, gemeprost, metenoprost, sulprostone and tiaprost can also be used. A particularly useful prostaglandin is prostaglandin E₁ (PGE₁) or its synthetic version, alprostadil. Esters of the prostaglandins, such as the methyl and ethyl esters, can also be used.

Suitable alpha-adrenoceptor blockers include phenoxybenzamine, dibenarnine, doxazosin, terazosin, phentolamine, tolazoline, prazosin, trimazosin, alfuzosin, tamsulosin and indoramin.

Ergot alkaloids include ergotamine and ergotamine analogs, e.g., acetergamine, brazergoline, bromerguride, cianergoline, delorgotrile, disulergine, ergonovine maleate, ergotamine tartrate, etisulergine, lergotrile, lysergide, mesulergine, metergoline, metergotamine, nicergoline, pergolide, propisergide, proterguride and terguride. A particularly effective alkaloid is yohimbine hydrochloride.

Non-specific phosphodiesterase inhibitors that can be incorporated into the condom include theophylline, IBMX, pentoxifylline and papaverine, and direct vasodilators such as hydralazine. Papaverine is particularly useful either alone or in combination with phentolamine.

Examples of type III phosphodiesterase inhibitors that may be used include bipyridines such as milrinone and amirinone, imidazolones such as piroximone and enoximone, dihydropyridazinones such as imazodan, 5-methyl-imazodan, indolidan and 1CI1118233, quinolinone compounds such as cilostamide, cilostazol and vesnarinone, and other molecules such as bemoradan, anergrelide, siguazodan, trequinsin, pimobendan, SKF-94120, SKF-95654, lixazinone and isomazole.

Examples of suitable type IV phosphodiesterase inhibitors include rolipram and rolipram derivatives such as RO20-1724, nitraquazone and nitraquazone derivatives such as CP-77059 and RS-25344-00, xanthine derivatives such as denbufylline and ICI63197, and other compounds such as EMD54622, LAS-31025 and etazolate.

Examples of type V phospodiesterase inhibitors include zaprinast, MY5445, dipyridamole, vardenafil, and sildenafil. Other suitable type V phosphodiesterase inhibitors are disclosed in PCT Publication Nos. WO 94/28902 and WO 96/16644. A particularly useful type V phosphodiesterase inhibitor is sildenafil. Still other type V phosphodiesterase inhibitors useful in conjunction with the present invention include: IC-351 (ICOS); 4-bromo-5-(pyridylmethylamino)-6-[3-(4-chlorophenyl)propoxy]-3(2H)pyridazinone; 1-[4-[(1,3-benzodioxol-5-ylmethyl)amiono]-6-chloro-2-quinazolinyl]-4-piperidine-carboxy lic acid, monosodium salt; (+)-cis-5,6a,7,9,9a-hexahydro-2-[4-(trifluoromethyl) -phenylmethyl-5-methyl-cyclopent-4,5]imidazo(2,1-b]purin-4(3H)one, furazlocillin; cis-2-hexyl-5-methyl-3,4,5,6a,7,8,9,9a-octahydrocyclopent[4,5]imidazo[2,1-b]purin-4-one; 3-acetyl-1-(2-chlorobenzyl)-2-propylindole-6-carboxylate; 4-bromo-5-(3-pyridylmethylamino)-6-(3-(4-chlorophenyl) propoxy)-3-(2H)pyridazinone; 1-methyl-5-(5-morpholinoacetyl-2-n-propoxyphenyl)-3-n-propyl-1,6,-dihydro-7H-pyrazolo (4,3-d)pyrimidin-7-one; 1-[4-[1,3-benzodioxol-5-ylmethyl)amino]-6-chloro-2-quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Scherin Plough); GF-196960 (Glaxo Wellcome); and Sch-51866.

Other compounds that can be used include nimodipine, pinacidil, cyclandelate, isoxsuprine, chloromazine, haloperidol, Rec15/2739 and trazodone, as well as anti-hyertensive agents including diazoxide, hydralazine and minoxidil.

The active compound or compounds optionally together with skin penetration enhancers may be applied direct to the appropriate region of the condom or as a composition dispersed or dissolved in a suitable carrier media, for example a gel carrier comprising a liquid medium and a thickening agent. According to another aspect of the invention, therefore, a composition for application to the external surface of a condom after unrolling on an erect penis comprises a vasodilator active compound and a carrier material.

In yet another embodiment, the invention provides a method of treating female sexual dysfunction, the method comprising the use during sexual intercourse of a condom having applied to the external surface a vasodilator active compound.

A condom according to the invention may also have a vasodilator active compound applied to the interior surface, in order to assist in maintaining an erection of the penis and, thus, providing for additional condom safety in prevention of premature and involuntary unsheathing of the condom from an otherwise partially-erect or flaccid penis. A compound applied to the interior surface may be localised at or towards the head end and may be the same or a different active compound to that applied to the external surface.

Embodiments of the invention will now be described by way of example only.

### Example 1

A condom having a textured portion towards the open end was made by dipping a former into compounded natural rubber latex, the former having a series of groves etched into its surface so as to form a series of ribs near the open end of the condom. After the condom was removed from the former it was mounted on a mandrel and a thin coating of a plasticised thermoplastic elastomer (a tri-block copolymer of styrene and butadiene) dissolved in a suitable solvent containing glycerol trinitrate absorbed onto lactose was applied by roller to the ribbed portion of the condom. The solvent was evaporated to leave the coating on the textured portion. The condoms were then rolled off the mandrel and lubricated and packed as normal. A water-based lubricant, which is immiscible with glycerol trinitrate, was selected. The presence of the lactose enhanced the texture of the ribs and acted as a source of glycerol trinitrate.

### Example 2

A 10 mm wide strip of a plasticised thermoplastic elastomer containing glycerol trinitrate dissolved in monopropylene glycol was extruded onto release paper using a die to form a profile having a number of raised ribs. The extruded strip, still on the release paper, was cut into a number of strips, the length of each strip being the circumference of the condom. Standard, parallel sided, non-ribbed condoms were mounted onto suitable mandrels and the strips were wrapped around the condoms near to the open end. The strips were then made to adhere to the condom by applying a heated roller and the release paper was removed. The condoms were then rolled, lubricated and packed as normal.

### Example 3

A standard, un-ribbed condom was mounted on a mandrel and a thin film of a plasticised thermoplastic elastomer dissolved in a suitable solvent was sprayed onto the condom in a narrow band close to its open end. The elastomer contained glycerol trinitrate dissolved in monopropylene glycol. The solvent was removed by evaporation and the mandrel was brought into contact with a hot embossed roller so that the roller pressed onto the band. The mandrel was rotated so as to leave an embossed pattern in the band. The condom was removed from the mandrel and then lubricated and packed.

## Claims

1. A condom having applied to its external surface a vasodilator active compound and being coated with a lubricant, **characterised in that** the vasodilator active compound is disposed on the external condom surface in a form or within a composition which is immiscible with the lubricant.

2. A condom according to claim 1, in which the active compound is applied as a composition which includes a carrier material with which the vasodilator compound is miscible but which will release the vasodilator active compound when in contact with body tissue.

3. A condom according to claim 1 or claim 2, in which the lubricant is buffered to a pH between 3 and 5.

4. A condom according to any preceding claim, in which the condom includes a textured or undulating region to the external surface.

5. A condom according to claim 4, in which the textured or undulating region extends at least towards the open end of the condom and incorporates or includes the vasodilator active compound.

6. A condom according to claim 5, in which the textured or undulating region is formed from one or more layers of material including the material from which the condom itself is formed, the material of at least one such layer being miscible with the vasodilator and allowing the vasodilator to be absorbed by skin or tissue when brought in contact with the condom.

7. A condom according to any preceding claim, in which the vasodilator active compound is selected from nitrates, long and short acting alpha-adrenoceptor blockers, ergot alkaloids, anti-hypertensives, the prostaglandins and phosphodiesterase inhibitors optionally in combination with a skin penetration enhancer.

8. A condom according to claim 7, in which the vasodilator active compound comprises an organic nitrate applied as a layer or coating to the condom external surface.

9. A condom according to claim 8, in which the organic nitrate layer or coating is applied in a polar elastomer in solution, in the form of an aqueous dispersion of latex or by a hot melt or reactive process.

10. A condom according to any preceding claim, in which the active compound optionally together with a skin penetration enhancer is applied to the condom as a composition dispersed or dissolved in a gel carrier comprising a liquid medium and a thickening agent.

## Patentansprüche

1. Kondom, auf dessen Außenfläche ein Vasodilator-Wirkstoff aufgetragen und der mit einem Gleitmittel beschichtet ist, **dadurch gekennzeichnet, dass** der Vasodilator-Wirkstoff auf der Außenfläche des Kondoms in einer Form oder in einer Zusammensetzung aufgetragen ist, die mit dem Gleitmittel nicht mischbar ist.

2. Kondom nach Anspruch 1, bei dem de Wirkstoff als Zusammensetzung aufgetragen ist, die einen Träger aufweist, mit dem die Vasodilator-Verbindung mischbar ist, die aber in Kontakt mit Körpergewebe den Vasodilator-Wirkstoff freigibt.

3. Kondom nach Anspruch 1 oder 2, bei dem das Gleitmittel auf einem pH-Wert zwischen 3 und 5 gepuffert ist.

4. Kondom nach einem der vorgehenden Ansprüche, dessen Außenfläche einen texturierten oder gewellten Bereich aufweist.

5. Kondom nach Anspruch 4, bei dem der texturierte oder gewellte Bereich sich mindestens zum offenen Ende des Kondoms erstreckt und den Vasodilator-Wirkstoff enthält bzw. aufweist.

6. Kondom nach Anspruch 5, bei dem der texturierte oder gewellte Bereich aus einer oder mehr Schichten gebildet ist, die das Material aufweisen, aus dem das Kondom selbst ausgebildet ist, wobei das Material mindestens einer solcher Schichten mit dem Vasodilator mischbar ist und erlaubt, dass der Vasodilator von Haut oder Gewebe absorbiert wird, die bzw. das im Kontakt mit dem Kondom gebracht wird.

7. Kondom nach einem der vorgehenden Ansprüche, bei dem der Vasodilator-Wirkstoff aus der Gruppe der Nitrate, lang- und kurzwirkenden Alpha-Adrenoceptor-Blocker, Ergotalkaloide, Antihypertensive, Prostaglandine und Phosphodiesterasehemmer optional in Kombination mit einer die Hauteindringung verbessernden Substanz gewählt ist.

8. Kondom nach Anspruch 7, bei dem der Vasodilator-Wirkstoff ein organisches Nitrat aufweist, das als Lage oder Beschichtung auf die Außenfläche des Kondoms aufgetragen ist.

9. Kondom nach Anspruch 7, bei dem der Vasodilator-Wirkstoff ein organisches Nitrat aufweist, das als Lage oder Beschichtung auf die Außenfläche des Kondoms aufgetragen ist.

10. Kondom nach einem der vorgehenden Ansprüche, bei dem der Wirkstoff optional zusammen mit einer die Hauteindringung verbessernden Substanz auf das Kondom als Zusammensetzung aufgetragen ist, die in einem Trägergel dispergiert oder gelöst ist, das einflüssiges Medium und ein Eindickungsmittel aufweist.

## Revendications

1. Préservatif à la surface externe duquel est appliqué un composé à action vasodilatatrice et qui est revêtu d'un lubrifiant, **caractérisé en ce que** le composé à action vasodilatatrice est placé à la surface externe du préservatif sous une forme ou dans une composition qui est immiscible avec le lubrifiant.

2. Préservatif selon la revendication 1, dans lequel le composé actif est appliqué sous forme d'une composition qui contient une matière formant un véhicule avec lequel le composé vasodilatateur est miscible, mais qui libère le composé à action vasodilatatrice lorsqu'il est au contact d'un tissu corporel.

3. Préservatif selon la revendication 1 ou 2, dans lequel le lubrifiant est tamponné à un pH compris entre 3 et 5.

4. Préservatif selon l'une quelconque des revendications précédentes, dans lequel le préservatif possède une région texturée ou ondulée vers la surface externe.

5. Préservatif selon la revendication 4, dans lequel la région texturée ou ondulée s'étend au moins vers l'extrémité ouverte du préservatif et incorpore ou comprend le composé à action vasodilatatrice.

6. Préservatif selon la revendication 5, dans lequel la région texturée ou ondulée est formée d'une ou plusieurs couches d'un matériau comprenant le matériau dont le préservatif est lui-même formé, le matériau d'au moins une telle couche étant miscible avec le composé vasodilatateur et permettant au composé vasodilatateur d'être absorbé par la peau ou le tissu lorsqu'il est mis au contact du préservatif.

7. Préservatif selon l'une quelconque des revendications précédentes, dans lequel le composé à action vasodilatatrice est sélectionné parmi les nitrates, les agents bloquants alpha-adrénocepteurs à action longue et courte, les alcaloïdes d'ergot, les agents anti-hypertenseurs, les prostaglandines et inhibiteurs de phosphodiestérase, éventuellement en combinaison avec un agent d'accentuation de la pénétration de la peau.

8. Préservatif selon la revendication 7, dans lequel le composé à action vasodilatatrice comprend un nitrate organique appliqué sous forme d'une couche ou d'un revêtement à la surface externe du préservatif.

9. Préservatif selon la revendication 8, dans lequel la couche ou le revêtement de nitrate organique est appliqué dans un élastomère polaire en solution, sous forme d'une dispersion aqueuse d'un latex, ou par un procédé par thermofusion ou réaction.

10. Préservatif selon l'une quelconque des revendications précédentes, dans lequel le composé actif, éventuellement avec un agent qui accentue la pénétration de la peau, est appliqué au préservatif sous forme d'une composition qui est dispersée ou dissoute dans un véhicule sous forme d'un gel comprenant un milieu liquide et un agent épaississant.
